# EUROPEAN PATENT SPECIFICATION

(11) **EP 4 504 144 B1**
(45) Date of publication and mention of the grant of the patent: **18.03.2026**
(21) Application number: 23717880.1
(22) Date of filing: 05.04.2023
(51) Int. Cl.: A61K 9/00, A61K 9/70

(54) **ORAL THIN FILM**
ORALER DÜNNFILM
FILM MINCE ORAL

(30) Priority: 05.04.2022 EP 22166845
(43) Date of publication of application: 12.02.2025
(73) Proprietor: LTS LOHMANN Therapie-Systeme AG, 56626 Andernach (DE)
(72) Inventor: BAUER, Marius, 56626 Andernach (DE); EMGENBROICH, Marco, 53359 Rheinbach (DE)
(74) Representative: Meissner Bolte Partnerschaft mbB
(86) International application number: PCT/EP2023/058905
(87) International publication number: WO 2023/194417

(56) References cited:
- EP-A1- 3 895 691
- US-B2- 10 398 746
- FAISAL WALEED ET AL: "Taste Masking Approaches for Medicines", CURRENT DRUG DELIVERY, BENTHAM SCIENCE PUBLISHERS, HILVERSUM, NL, vol. 15, no. 2, 14 February 2018 (2018-02-14), pages 167 - 185, XP002805525, ISSN: 1567-2018, DOI: 10.2174/1567201814666171013145958

## Description

The present invention relates to an oral thin film containing at least one active pharmaceutical agent, a method for preparing same, and the use of such an oral thin film as a medicament, especially for use as an emergency contraceptive.

Oral thin films are thin films containing at least one pharmaceutically active agent that are placed directly in the oral cavity or on the oral mucosa and dissolve or disintegrate there and in doing so release the active agent which can then be absorbed transmucosally or, after swallowing, gastrointestinally. The active agent may be dissolved, emulsified or dispersed in the film. An example of an oral thin film comprising ulipristal acetate is shown in EP 3 895 691 A1.

As explained in greater detail further below, the oral thin film according to the present invention contains ulipristal acetate as pharmaceutically active agent and is preferably used as emergency contraceptive.

When administering some pharmaceutically active ingredients with an oral thin film, it may be desired that the pharmaceutically active ingredients are rapidly released, which is usually achieved by a rapid disintegration time of the oral thin film.

In other cases, however, it may be desired when administering some pharmaceutically active ingredients with an oral thin film, that the pharmaceutically active ingredients are released slowly and/or that the release of the pharmaceutically active ingredients is delayed to prevent or at least minimize transmucosal in favor of gastrointestinal absorption.

This is usually achieved by increasing the thickness and/or the area weight of the oral thin film in order for the oral thin film to disintegrate slower, which results in a slower release of the pharmaceutically active ingredients.

However, in certain applications, thick oral thin films are not desired and it is also not desired to increase or delay the disintegration time of an oral thin film after oral application.

Hence there was a need for an oral thin film, with which an active pharmaceutical ingredient can be administered, wherein the oral thin film has a rather rapid disintegration time, preferably of less than 30 seconds, however, wherein the release of the active pharmaceutical ingredient is uncoupled from the disintegration time and can be delayed independently from the disintegration time.

In addition, it should be possible to produce such an oral thin film as easily and economically as possible.

It was furthermore a specific objective to provide an oral thin film, in particular an oral thin film, which is preferably non-mucoadhesive, comprising ulipristal acetate as active pharmaceutical ingredient which is bioequivalent to the commercially available EllaOne^{®} tablets. Therefore, the film has to exhibit disintegration and release characteristics which prevents or at least minimizes transmucosal absorption and allows for gastrointestinal absorption of ulipristal acetate.

Thus, it was a specific objective to develop an oral thin film that has a delayed in vitro drug release and thus has a higher comparability to the in vitro release of the marketed Ulipristal Acetate tablet EllaOne^{®} than previously known ulipristal acetate oral thin films. At the same time, the oral thin film should still exhibit a rapid disintegratation, so that it can be swallowed quickly and no unpleasant sensation occurs in the mouth (e.g. triggered by a bitter taste of the active ingredient or a foreign body sensation due to residues of the film) and transmucosal absorption is prevented.

Normally, delayed release of active ingredients in oral thin films can be achieved by reducing the disintegration time. This can be done by increasing the thickness or area weight, or by selecting polymers with lower solubility in water or saliva. This causes the oral thin film to disintegrate more slowly, resulting in a slower release of the active ingredient. However, since the oral thin film should not have a too high disintegration time, this approach was not suitable.

Based on the principles of the long known commercially marketed product "Theraflu Thin Strips Long acting cough", the possibility of binding ulipristal acetate to an ionic component of the film matrix by ion-pair bonding was taken into account, so that the film disintegrates quickly but the active ingredient is released from the ion-pair bond only after a longer period of time and can therefore be absorbed with a delay.

In the case of "Theraflu Thin Strips Long acting cough", the ion-pair bond is created between the cationic group of the basic active ingredient dextromethorphan HBr and the anionic carboxylic acid groups of an ion-exchange resin (e.g. Amberlite IRP 64). The formation of the ion-pair bond reduces the bitter taste of the dextromethorphan, since the cationic tertiary amine, which is responsible for the bitter taste, is no longer freely present in the mouth. A side effect is that the ion-pair bond is not completely broken by the acidic stomach pH until after swallowing in the stomach, which results in delayed drug release.

In initial trials, anionic ion exchange resin Amberlite IRP 64 was tested for ion pair bonding with the cationic tertiary amine of ulipristal acetate. Due to the high required dose of preferably 30 mg of ulipristal acetate, which must be present only in suspension in the film, it was not possible to use the powdered Amberlite IRP 64 as a counter ion. Amberlite IRP 64 is in powder form and was insoluble in the solvent used, which was water. Since normally the active ingredient and the corresponding ion exchange resin are used in a ratio of about 1:1, another 30 mg of Amberlite IRP 64 was present undissolved in each individual dose in addition to the 30 mg of undissolved ulipristal acetate already present. This resulted in a total of 60 mg of powdered solids to be incorporated into an OTF with a target total weight of 90 mg. Due to the high solids content, it was not possible to produce a film with sufficient flexibility for commercial use.

Thus, it was another objective of the present invention to overcome the above-mentioned disadvantages of the known market products.

The above mentioned objectives were surprisingly solved by an oral thin film according to claim 1 comprising a polymer matrix, which comprises an active pharmaceutical ingredient, a first polymer and a second polymer, wherein the first polymer is different from the second polymer and wherein the active pharmaceutical ingredient comprises ulipristal acetate and the second polymer is an anionic.

Preferred embodiments are described in the dependent claims.

The advantage of using such a combination of polymers and active pharmaceutical ingredients is that, compared to water-insoluble ion exchange resins, higher amounts of active ingredient can be bound or incorporated without the film losing flexibility.

The use of these combinations also makes it possible to keep the area weight low, which has a positive effect on the disintegration time, so that disintegration times of less than 30 seconds can be achieved.

Using an ion exchange resin instead of the polymers would only be possible by greatly increasing the amount of film-forming polymers in the formulation, which, if the film size should remain the same, requires increasing the area weight, which in turn would lead to longer disintegration times.

Although the findings and the invention as claimed relate to an oral thin film with ulipristal acetate, the general concept of a combination of a cationic active pharmaceutical ingredient and an anionic polymer, or a combination of an anionic active pharmaceutical ingredient and a cationic polymer can be applied to all possible active pharmaceutical ingredients.

Depending on the basis of the film composition, the use of the anionic or the cationic polymers for sustained release is also not limited to water-soluble polymers but can be transferred to non-water-soluble polymers.

The ensuing description provides some embodiment(s) of the invention, and is not intended to limit the scope, applicability or configuration of the invention or inventions. Various changes may be made in the function and arrangement of elements as long as they stay within the scope of the invention as set forth in the claims.

The terminology used in the description of the disclosure herein is for the purpose of describing particular embodiments only and is not intended to be limiting of the subject matter. As used in this description and the appended claims, the singular forms "a", "an" and "the" are intended to include the plural forms as well, unless the context clearly indicates otherwise. It will also be understood that the term "and/or" as used herein refers to and encompasses any and all possible combinations of one or more of the associated listed items. It will be further understood that the terms "includes," "including," "comprises," and/or "comprising," can also be understood as "consisting of" when used in this specification.

It will also be understood that, although the terms first, second, etc. may be used herein to describe various elements, these elements should not be limited by these terms. These terms are only used to distinguish one element from another. For example, a first object or step could be termed a second object or step, and, similarly, a second object or step could be termed a first object or step. The first object or step, and the second object or step, are both objects or steps, respectively, but they are not to be considered the same object or step.

The present invention provides an oral thin film comprising a polymer matrix, which comprises an active pharmaceutical ingredient, a first polymer and a second polymer, wherein the first polymer is different from the second polymer and wherein the active pharmaceutical ingredient comprises ulipristal acetate and the second polymer is an anionic.

The polymer matrix may also be understood as a matrix layer or a polymer layer.

In the context of the invention, a cationic polymer refers to a polymer with a positive charge, in particular net charge, at a pH of 6 to 9 and a temperature of 20°C.

The cationic polymer preferably has a pKₐ value of 8.5-10.5

In the context of the invention, an anionic polymer refers to a polymer with a negative charge, in particular net charge, at a pH of 3.5-to 8 and a temperature of 20°C.

The anionic polymer preferably has a pKₐ value of 3.5-6.5

In the context of the invention, a cationic active pharmaceutical ingredient refers to an active pharmaceutical ingredient with a positive charge, in particular net charge, at a pH of 6 to 8 and a temperature of 20°C.

The cationic active pharmaceutical ingredient preferably has a pKₐ value of 8.5-10.5

In the context of the invention, an anionic active pharmaceutical ingredient refers to an active pharmaceutical ingredient with a negative charge, in particular net charge, at a pH of 6 to 8 and a temperature of 20°C.

The anionic active pharmaceutical ingredient preferably has a pKₐ value of 3.5-6.5

The first and the second polymer are different.

While the first polymer is preferably present to form the scaffold of the polymer matrix (film forming polymer), the second polymer is preferably present to form an ion pairing with the active pharmaceutical ingredient and thus acts as release retardant.

Therefore, it is preferred that the amount of the first polymer in the polymer matrix is higher than the amount of the second polymer in the polymer matrix.

In a preferred embodiment, the first polymer is a water-soluble polymer.

By water-soluble is preferably meant a solubility of greater than 10 g/L, preferably greater than 50 g/L and in particular 100 g/L in water at 25°C.

In a preferred embodiment, the first polymer is an uncharged, with film forming capabilities and preferably a good solubility in water or aqueous medium.

Water-soluble polymers, which shall herein also encompass water-swellable polymers, comprise chemically very different, natural or synthetic polymers whose common feature is their solubility or swelling capacity in water or aqueous media. The prerequisite is that these polymers have a sufficient number of hydrophilic groups for water solubility and are not crosslinked. The hydrophilic groups can be non-ionic.

By water-soluble is preferably meant a solubility of greater than 10 g/L, preferably greater than 50 g/L and in particular 100 g/L in water at 25°C.

Preferably, the first polymer is a water-soluble or water-swellable polymer selected from the group consisting of starch and starch derivatives, dextrans, cellulose derivatives, hydroxypropyl cellulose, hydroxyethyl cellulose, hydroxypropylmethyl cellulose, hydroxypropyl ethyl celluloseor propyl cellulose, polyvinylpyrrolidones, polyvinyl alcohols, polyethylene oxide polymers, polyacrylamides, polyethylene glycols, gelatine, collagen, pullulan, tragacanth, arabinogalactan, galactomannan, agar, agarose, carrageenan and/or natural gums.

Herein, polyvinyl alcohol is preferred.

Further, a mixture of two or more polyvinyl alcohols is preferred.

Commercially customary polyvinyl alcohols, which are offered in the form of white-yellow powders or granules, generally have a degree of hydrolysis of 98 to 99 or 87 to 89 mol %, that is to say also contain a residual content of acetyl groups. The polyvinyl alcohols are usually characterized by the manufacturer by a specification of the degree of polymerization of the starting polymers or the mean molecular weight, the degree of hydrolysis, the saponification number or the solution viscosity.

The oral thin film according to the invention is preferably characterized in that the at least one polyvinyl alcohol comprises a polyvinyl alcohol with a mean molecular weight of approximately 25,000 to approximately 250,000 g/mol.

The oral thin film according to the invention is preferably characterized in that the at least one polyvinyl alcohol comprises a polyvinyl alcohol with a mean molecular weight of approximately 25,000 to approximately 35,000 g/mol and/or a polyvinyl alcohol with a mean molecular weight of approximately 200,000 to 210,000 g/mol.

According to the present invention, polyvinyl alcohols with a mean molecular weight of approximately 31,000 (4-88) to approximately 205,000 (40-88) g/mol are especially suitable.

According to the present invention, polyvinyl alcohols with a mean molecular weight with approximately 31,000 (4-88) to approximately 205,000 (40-88) g/mol are especially suitable.

Preferred are also mixtures of the before described 4-88 and 40-88, preferably in a weight ratio of 20:1 to 5:1, or 15:1 to 7:1.

In a preferred embodiment, the active pharmaceutical ingredient comprises a rather non-water soluble active pharmaceutical ingredient, preferably with a solubility in water of less than 100 g/L, preferably less than 50 g/L and in particular less than 10 g/L (all at 20°C).

In a preferred embodiment, the active pharmaceutical ingredient comprises a rather lipophilic active pharmaceutical ingredient, preferably with a logP of more than 1, preferably more than 2, in particular more than 3 and especially more than 4, but less than 10, or less than 8, preferably less than 7 and especially less than 6.

In a preferred embodiment, the active pharmaceutical ingredient comprises a rather lipophilic active pharmaceutical ingredient, preferably with a logP between 3 and 5, preferably between 3.6 and 4.7.

The active pharmaceutical ingredient comprises ulipristal acetate.

Ulipristal acetate is 7α-acetoxy-11α-(4-N,N-dimethylaminophenyl)-19-norpregna-4,9-dien-3,20-dion) with the following chemical formula:

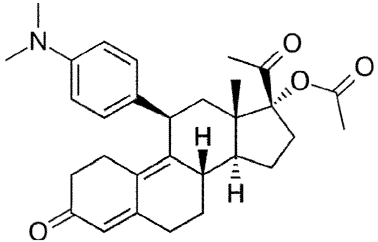

Ulipristal acetate is a well-known emergency contraceptive ("morning-after pill").

It is administered as a tablet ("EllaOne^{®}") containing 30 mg micronized ulipristal acetate and lactose monohydrate, povidone, croscarmellose sodium and magnesium stearate as further ingredients. EllaOne^{®} was approved in the European Union in 2009.

Tablets like EllaOne^{®} are usually taken with water to ease swallowing. In regions where there is no quick access to clean drinking water, taking tablets might thus be challenging, especially for certain patient groups having difficulties with swallowing medications i.e. suffering from dysphagia. Administration of oral thin films, which quickly dissolve upon application in the oral cavity, do not require additional water and are therefore advantageous.

At a pH between 6 to 8, ulipristal acetate is present in protonated, positively charged form.

In a preferred embodiment, the second polymer is a non water-soluble polymer, which means that the solubility in water is less than 10 g/L at 25°C.

The second polymer is an anionic polymer, which comprises a polymer backbone and side chains. These side chains preferably comprise anionic groups, preferably at a pH of 6 to 8, such as sulfones, carboxylates and/or phosphates.

In particular, the anionic polymer comprises polyacrylic acid, a carboxylate modified cellulose, pectin and/or alginate.

Particularly preferred are carboxylate modified celluloses, which encompass celluloses that carry carboxylate groups, preferably as alkali metal salts.

In particular preferred are alkali salts of carboxyalkyl celluloses, such as carboxymethyl cellulose or carboxyethyl cellulose.

Particularly preferred are sodium salts of carboxymethyl cellulose or carboxyethyl cellulose.

Especially preferred is sodium carboxymethyl cellulose.

A suitable sodium carboxymethyl cellulose is known under the name "Blanose 7LP".

Another preferred anionic polymer comprises polyacrylic acid.

Suitable polyacrylic acids are known under the names "Carbopol 934" or "Carbopol 971". Carbopol 934 has a molecular weight of about 3 Mio g/mol, while Carbopol 971 has a molecular weight of about 1.25 Mio g/mol.

In a preferred embodiment, the combination of the cationic active pharmaceutical ingredient and the anionic polymer comprises sodium carboxymethyl cellulose and ulipristal acetate.

In another preferred embodiment, the combination of the cationic active pharmaceutical ingredient and the anionic polymer comprises polyacrylic acid and ulipristal acetate.

In a preferred embodiment, the combination of the cationic active pharmaceutical ingredient and the anionic polymer comprises sodium carboxymethyl cellulose and ulipristal acetate and the first polymer comprises polyvinyl alcohol or a mixture of two polyvinyl alcohols.

In another preferred embodiment, the combination of the cationic active pharmaceutical ingredient and the anionic polymer comprises polyacrylic acid and ulipristal acetate and the first polymer comprises polyvinyl alcohol or a mixture of two polyvinyl alcohols.

Preferably, the second polymer in a cationic polymer, which comprises a polymer backbone and side chains. These side chains preferably comprise cationic groups, preferably at a pH of 6 to 8, such as amines.

Further suitable cationic polymers comprise polyethylene imine, chitosan and/or, poly(L-lysine).

In a preferred embodiment, the first polymer is contained in the polymer matrix in an amount of 10 to 60 wt.-%, preferably 20 to 40 wt.-%, based on the total weight of the polymer matrix.

In another preferred embodiment, the first polymer comprises a mixture of two polymers that are in sum contained in the polymer matrix in an amount of 10 to 60 wt.-%, preferably 20 to 40 wt.-%, based on the total weight of the polymer matrix.

In a preferred embodiment, the first polymer comprises polyvinyl alcohol that is contained in the polymer matrix in an amount of 10 to 60 wt.-%, preferably 20 to 40 wt.-%, based on the total weight of the polymer matrix.

In another preferred embodiment, the first polymer comprises a mixture of two polyvinyl alcohols that are in sum contained in the polymer matrix in an amount of 10 to 60 wt.-%, preferably 20 to 40 wt.-%, in particular 24 to 32 wt.-%, based on the total weight of the polymer matrix.

In a preferred embodiment, the second polymer is contained in the polymer matrix in an amount of 1 to 30 wt.-%, preferably 2 to 15 wt.-%, in particular 4 to 8 wt.-%, based on the total weight of the polymer matrix.

In another preferred embodiment, the second polymer comprises a polyacrylic acid and is contained in the polymer matrix in an amount of 1 to 30 wt.-%, preferably 2 to 15 wt.-%, in particular 4 to 8 wt.-%, based on the total weight of the polymer matrix.

In another preferred embodiment, the second polymer comprises sodium carboxymethyl cellulose and is contained in the polymer matrix in an amount of 1 to 30 wt.-%, preferably 2 to 15 wt.-%, in particular 4 to 8 wt.-%, based on the total weight of the polymer matrix.

In a preferred embodiment, the weight ratio of the first polymer to the second polymer is from 15:1 to 2:1, preferably from 12:1 to 2:1, in particular from 10:1 to 2:1, more particular from 8:1 to 2:1, or about 7:1 to 2:1, or about 6:1 to 2:1, or about 5:1 to 2:1, or about 4:1 to 2:1, or about 3:1 to 2:1.

In particular, the preferred weight ratio of the first polymer to the second polymer is from 7:1 to 2:1 or from 7:1 to 2.5 to 1.

Another preferred weight ratio of the first polymer to the second polymer is from 4.5:1 to 3.5 to 1 or from 7.5 to 1 to 6.5 to 1.

In a preferred embodiment, the active pharmaceutical ingredient is contained in the polymer matrix in an amount of 0.1 to 50 wt.-%, preferably 0.5 to 50 wt.-%, preferably 1 to 50 wt.-%, preferably 5 to 50 wt.-%, preferably 10 to 50 wt.-%, preferably 20 to 50 wt.-%, preferably 35 to 45 wt.-%, in particular from 37 to 40 wt.-% or from 40 to 44 wt.-% or from 37 to 44 wt.-%, based on the total weight of the polymer matrix.

In a preferred embodiment, the active pharmaceutical ingredient comprises ulipristal acetate that is contained in the polymer matrix in an amount of 20 to 50 wt.-%, preferably 35 to 45 wt.-%, in particular from 37 to 40 wt.-% or from 40 to 44 wt.-% or from 37 to 44 wt.-%, based on the total weight of the polymer matrix.

In a preferred embodiment, the active pharmaceutical ingredient comprises ulipristal acetate that is contained in a total amount of 0.1 to 100 mg, preferably 1 to 100 mg, preferably 10 to 100 mg, preferably of 10 to 50 mg, in particular of 20 to 40 mg, especially in about 30 mg in the oral thin film.

The oral thin film according to the invention is also preferably characterised in that the polymer matrix further comprises at least one auxiliary substance selected from the group comprising colouring agents, flavourings, sweeteners, softeners, taste-masking agents, emulsifiers, enhancers, pH regulators, humectants, preservatives and/or antioxidants.

Each of these auxiliary substances is preferably contained in each case in an amount of 0.1 to 15 wt.%, preferably of 0.1 to 10 wt.%, or of 0.1 to 5 wt.%, in relation to the total weight of the polymer matrix.

The oral thin film according to the present invention is not subjected to any limitations in respect of its structure.

The oral thin film according to the invention can thus be provided in the form of a single-layer oral thin film and thus can consist merely of the polymer matrix as defined above.

In another embodiment, the oral thin film according to the invention can be provided in the form of a multi-layer oral thin film and thus can contain further layers in addition to the polymer matrix as defined above.

This plurality of layers can be laminated directly on top of one another or can be connected to an adhesive layer arranged in between.

An adhesive layer is understood to mean a layer that can act as an adhesive, as defined in DIN EN 923:2016-03. A non-adhesive layer therefore cannot act as an adhesive as defined above.

Especially, water-soluble adhesive layers as described in DE 10 2014 127 452 A1 are suitable as adhesive layers.

For example, buffer layers for setting a pH value or slowly dissolving or insoluble layers which protect the oral thin film against premature erosion can be provided as further layers.

Alternatively, further layers can be provided, which contain other active pharmaceutical ingredients or flavorings or taste-masking agents.

In one embodiment the oral thin film according to the present invention is characterized in that the polymer matrix is in the form of a smooth film. This means that the polymer matrix is for example not provided in the form of a foam.

A smooth film is preferably characterized in that a smooth film has a volume fraction of 0 to 5%, in relation to the total volume of the polymer matrix, of bubbles or cavities. The cavities are filled here preferably with air or a gas, preferably with an inert gas, especially preferably with nitrogen, carbon dioxide, helium or a mixture of at least two of these gases. The diameter of the bubbles or cavities generally lies in the range of 0.01 to 350 µm. The diameter of the bubbles or cavities especially preferably lies in the range of 10 and 200 µm.

In another embodiment the oral thin film according to the present invention is characterized in that the polymer matrix is in the form of a foamed (solidified) film having cavities.

Especially, the infiltration of water or saliva or other body fluids into the interior of the dosage form is facilitated by the cavities and the associated larger surface of the films, and therefore the disintegration of the dosage form is accelerated.

On the other hand, the wall thickness of said cavities is preferably low, since these represent, for example, solidified bubbles, and so these cavities dissolve or break down quickly.

A further advantage of this embodiment lies in the fact that, due to the formulation as a foam, a quicker drying can be provided than for a comparable, non-foamed composition, in spite of the comparatively high area density.

The oral thin film according to the present invention is preferably characterized in that the cavities are isolated from one another and are preferably provided in the form of bubbles, wherein the cavities are filled with air or a gas, preferably with an inert gas, especially preferably with nitrogen, carbon dioxide, helium or a mixture of at least two of these gases.

According to another embodiment the cavities are connected to one another preferably by forming a cohesive channel system penetrating the matrix.

Said cavities preferably have a volume fraction of 5 to 98%, preferably of 50 to 80%, in relation to the total volume of the polymer matrix. In this way, the advantageous effect of accelerating the dissolving of the polymer matrix is favorably influenced.

Furthermore, surface-active substances or surfactants can be added to the polymer matrix for foam formation or to the obtained foam before or after the drying in order to improve the stability of the foam before or after the drying.

A further parameter that influences the properties of the dosage form according to the invention is the diameter of the cavities or bubbles. The bubbles or cavities are preferably produced with the aid of a foaming machine, with which the diameter of the bubbles can be set within a wide range, almost arbitrarily. The diameter of the bubbles or cavities can thus lie in the range of 0.01 to 350 µm. The diameter preferably lies in the range of 10 and 200 µm.

The oral thin film according to the present invention preferably has an area of 0.5 cm² to 10 cm², especially preferably of 2 cm² to 8 cm² or of 6 cm² to 8 cm².

The area weight of the polymer matrix or of a further layer possibly provided is, in each case, preferably at least 10 g/m², more preferably at least 20 g/m² or at least 30 g/m² or most preferably 50 g/m², or less than or equal to 400 g/m², more preferably less than or equal to 350 g/m², or less than or equal to 300 g/m² or most preferably less than 250 g/m². The area weight is preferably 10 to 400 g/m², more preferably 20 to 350 g/m², or 30 to 300 g/m² and most preferably 50 to 250 g/m².

In another embodiment, the area weight is preferably 90 to 130 g/m², more preferably 95 to 125 g/m²,
Each of the provided polymer matrices or layers, especially the polymer matrix, preferably has in each case a layer thickness of preferably 10 µm to 500 µm, more preferably of 20 µm to 300 µm.

If the various layers, especially the polymer matrices, are present in the form of a solidified foam, it is thus preferred that each of the layers provided as a foam has, in each case, a layer thickness of preferably 10 µm to 3000 µm, more preferably of 90 µm to 2000 µm.

The oral thin film according to the present invention preferably has an (in vitro) disintegration time of less than 30 seconds, preferably of less than 27 seconds and in particular of 25 seconds or less, wherein the (in vitro) disintegration time is determined as described in the examples section.

In another embodiment, the oral thin film according to the present invention preferably has an (in vitro) disintegration time of less than 10 seconds, preferably of less than 7 seconds and in particular of 6 seconds or less, 5 seconds or less, or 3 seconds or less, wherein the (in vitro) disintegration time is determined as described in the examples section.

The oral thin film according to the present invention can be produced by conventional methods.

The above definitions in relation to the oral thin film apply similarly to the method according to the invention.

A method for producing the oral thin film according to the invention comprises the steps of:
a) preparing a solution, suspension and/or dispersion comprising an active pharmaceutical ingredient, a first polymer and a second polymer, wherein first polymer is different form the second polymer and wherein the active pharmaceutical ingredient and the second polymer are a combination of a cationic active pharmaceutical ingredient and an anionic polymer, or
   a combination of an anionic active pharmaceutical ingredient and a cationic polymer, in water, in an organic solvent and/or in a mixture thereof
b) casting or coating the solution, suspension and/or dispersion obtained in step a) on a support, coating liner or in a mould to spread the solution, suspension and/or dispersion, and
c) evaporating the solvent to obtain an oral thin film.

The solvent used comprises or consists of water or a mixture of water and one or more organic solvents, wherein the use of water is preferred. Examples for a suitable organic solvent are alcohols, in particular ethanol. The weight ratio of water to organic solvent, preferably ethanol, if used, may be e.g. in the range of 95/5 to 5/95, preferably in the range of 95/5 to 80/20.

In step a), the ingredients are mixed with each other to obtain a solution, suspension and/or dispersion. As some ingredient may be soluble in the chosen solvent while others are not, the obtained mixture in step a) might be a solution, suspension and/or dispersion at the same time.

The order for combining the ingredients is arbitrary.

In case of preparing a foam oral thin film, the suspension is generally foamed with a gas. Foaming is preferably carried out before pouring step b). Examples for a suitable gas for foaming are air, N₂, argon, or CO₂.

After evaporation of solvent a solid oral thin film is achieved.

The evaporation of the solvent is usually achieved by drying the spread solution, suspension and/or dispersion after step b) at temperatures up to 130 °C for about 5 min to 1h.

Drying conditions for solvent evaporation can be e.g. in the range of 40 to 130 °C, more preferably 50 to 80 °C, most preferably 50 to 75°C. It is also possible that drying is effected by applying a temperature gradient.

After solvent evaporation, the residual solvent content in the oral thin film obtained is preferably in the range of 0.2 to 10% by weight, preferably 0.8 to 6 % by weight, based on the total weight of the oral thin film. The residual solvent contained may be water or water and one or more organic solvents such as water and ethanol.

The films obtained can be cut and/or punched into pieces of desired dimensions.

The present invention further relates to an oral thin film obtained by the method as described above.

The above definitions in relation to the oral thin film apply similarly to the oral thin film obtained by the method as described above.

The present invention further relates to an oral thin film as described above for use as a medicament.

The present invention further relates to an oral thin film as described above for use as a contraceptive, in particular an emergency contraceptive, i.e. for use in the prevention of pregnancy after sex, in particular after unprotected sex. The oral film will be administered in the oral cavity. Addition of drinking water is not necessary. The active agent ulipristal acetate is mostly swallowed into the gastrointestinal tract in solid form so that bioequivalence to the approved ulipristal acetate tablet ("EllaOne^{®}") is accomplished.

The above definitions in relation to the oral thin film apply similarly to the medical uses of the oral thin film as described above.

The invention will now be explained more specifically with reference to the following examples, which are given for illustration of this invention and are not intended to be limiting thereof.

### Examples

Oral thin films of the compositions as set forth in Table 1 have been prepared according to the following protocol:

### Examples A1 to A3

Ulipristal acetate, the respective Carbopol, and sodium chloride were weighed in. Afterwards, the required total amount of water was added. The mixture was stirred for approx. 2h allowing the Carbopol to dissolve and to form ion pairs with Ulipristal acetate. Afterwards all remaining excipients were added in no particular order. The mass was stirred for further two hours.

Afterwards, the mass was coated on the polyethylene coated side of a paper intermediate release liner. Coating was performed using a coating knife with a defined coating gap to obtain the required final area weight.

The coated mass was dried with a maximum temperature of 60 °C for 30 min.

### Examples B1 to B2

Ulipristal acetate and NaCMC were weighed in.

Afterwards, the required total amount of water was added. The mixture was stirred for approx. 2h allowing NaCMC to dissolve and to form ion pairs with Ulipristal acetate. Afterwards all remaining excipients were added in no particular order. The mass was stirred for further two hours.

Afterwards, the mass was coated on the polyethylene coated side of a paper intermediate release liner. Coating was performed using a coating knife with a defined coating gap to obtain the required final area weight.

The coated mass was dried with a maximum temperature of 60 °C for 30 min.

Dried films were punched with a dye cut yielding with an area of 7.04 cm² and containing 30 mg ulipristal acetate.

**Table 1:**

| | | **REFERENCE** | **A1** | **A2** | **A3** | **B1** | **B2** |
|---|---|---|---|---|---|---|---|
| | | **wt.-%** | **wt.-%** | **wt.-%** | **wt.-%** | **wt.-%** | **wt.-%** |
| Ulipristal Acetate | API | 43.0 | 37.14 | 37.14 | 37.14 | 37.40 | 37.40 |
| PVA 4-88 | film forming polymer | 24.77 | 25.30 | 28.93 | 31.96 | 26.50 | 26.50 |
| PVA 40-88 | film forming polymer | 3.23 | 2.56 | 2.93 | 3.24 | 3.60 | 3.60 |
| Carbopol 934* | release retardant | --- | 10.50 | --- | --- | --- | --- |
| Carbopol 971** | release retardant | --- | --- | 7.50 | 5.00 | --- | --- |
| NaCMC (Blanose 7LP)*** | release retardant | --- | --- | --- | --- | 7.50 | 7.50 |
| NaCl | viscosity adjustment | --- | 3.50 | 2.50 | 1.66 | --- | --- |
| Span 83 | plasticizer | --- | 1.00 | 1.00 | 1.00 | --- | --- |
| Xylitol | sweetener | 2.00 | 2.00 | 2.00 | 2.00 | 2.00 | 2.00 |
| Tween 80 | plasticizer | 1.00 | 1.00 | 1.00 | 1.00 | --- | --- |
| Sorbitol | sweetener solubilizer | 9.00 | 4.50 | 4.50 | 4.50 | 6.00 | 6.00 |
| Sucralose | sweetener humectant | 1.00 | 1.00 | 1.00 | 1.00 | 1.00 | 1.00 |
| Glycerin | plasticizer | 9.00 | 4.50 | 4.50 | 4.50 | 9.00 | 9.00 |
| Menthol | flavor | 1.00 | 1.00 | 1.00 | 1.00 | 1.00 | 1.00 |
| Rice starch | solubilizer | 6.00 | 6.00 | 6.00 | 6.00 | 6.00 | 6.00 |
| **TOTAL** | | **100** | **100** | **100** | **100** | **100** | **100** |
| area weight | | 99.5 g/m² | 121 g/m² | 121 g/m² | 121 g/m² | 121 g/m² | 121 g/m² |
| Solvent | | H₂O | H₂O | H₂O | H₂O | H₂O | H₂O |
| pH value | | | | | | 4.5 | 7 |

| | | | | | | | |
|---|---|---|---|---|---|---|---|
| * polyacrylic acid ** polyacrylic acid *** sodium carboxymethyl cellulose | | | | | | | |

### In vitro release experiments:

In vitro release testing of the examples described above was performed using a Fiber Optic dissolution tester G00668. The detection is performed by UV/Vis

| | |
|---|---|
| Wavelength: | 310 nm (path length 10 mm) |
| Test media: | 900 ml of 0.1 M HCL, simulating the pH of an empty stomach |
| Stirring: | 50 rpm |
| Release: | Apparatus 1, USP Sinker (12 turns) |
| Release time: | 15 min |
| Testing intervals: | 12x every 5 seconds (scan rate of 4 per testing) |
| | 4x every 15 seconds (scan rate of 4 per testing) |
| | 13x every 60 seconds (scan rate of 4 per testing) |
| Temperature: | 37°C (±0,5°C) |
| n: | 3 (test per formulation) |

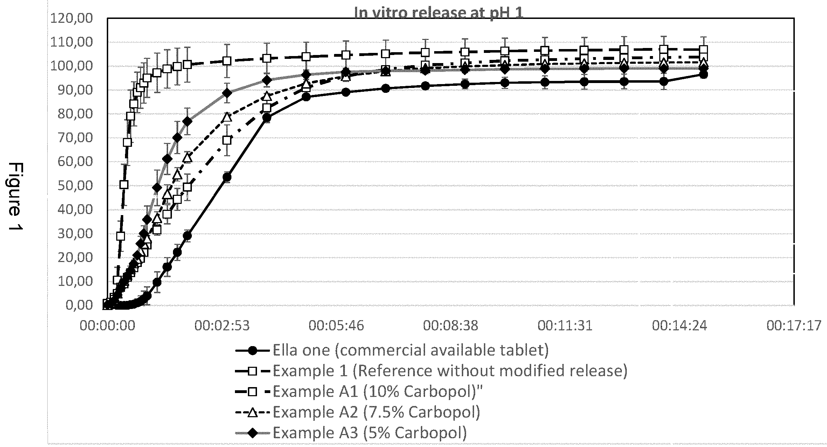

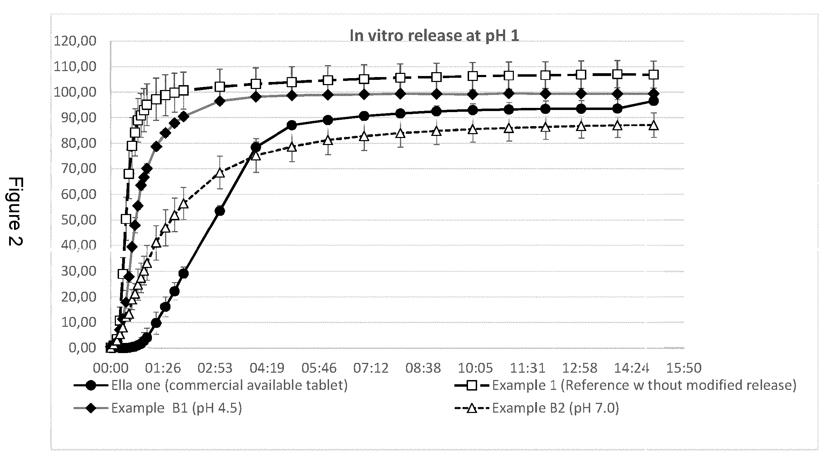

The results of the in vitro release experiments are given in Figure 1 and Figure 2.

The experiments show that the release of the ulipristal acetate can be delayed to a greater extent with increasing carbopol content, especially in comparison to the reference example without modified drug release.

The profile gets closer to the profile of EllaOne the higher the carbopol content is.

Higher concentrations of carbopol could be incorporated into the OTF, if necessary, to achieve an even greater delay in drug release. However, this was not tested, as the solubility of the OTF already decreases sharply from 10% carbopol, which would lead to a longer residence time in the mouth, which in turn could lead to an unpleasant feeling in the mouth.

Further, the experiments show that a delay in drug release could be achieved by adding 7.5% NaCMC (Blanose 7LP).

Incorporation of higher NaCMC concentrations would be possible to achieve an even higher delay in drug release. However, a higher NaCMC concentration would again lead to slower disintegration, which is not desired.

The experiments further show that the delayed drug release is most effective when the pH of the NaCMC formulation is not modified (Example B2).

The principle of ion-pair bonding in the formulations is based on the fact that the carboxylic acids of the polymers protonate the basic groups of the active ingredient (a tertiary amine), thus forming the ion bond.

In formulations A1 - A3, according to the current theory, the formulation already possessed a sufficient amount of acidic groups due to the presence of the carbopol, resulting in the protonation of the active ingredient, which in turn led to the ion-pair bond.

In formulation B1, however, NaCMC was used. A polymer that is present as a salt. Thus, parts of the carboxylic acid groups are present as deprotonated carboxylate ions. According to the current theory, the acidic groups/proton donors were thus missing in the system to protonate the ulipristal acetate and enable ion-pair bonding. For this reason, Formulation B1 was adjusted to a pH of 4.5 during preparation to allow ion pair formation between CMC and the drug.

However, the experiment showed that formulation B1 could only produce a negligible reduction in drug release.

In contrast, formulation B2 showed delayed drug release compared to formulation B1. In this formulation, the NaCMC (Blanose 7LP) was mixed together with ulipristal acetate in the formulation without any additional pH adjustment. The measured pH of the final mass was about 7.

A possible explanation for this observation is that in the case of NaCMC, in our particular case the Blanose 7LP, only parts of the carboxylic acid groups were "salinized" with sodium, while a large part of the carboxylic acids are still free.

Thus, despite its description as a sodium salt, Blanose 7LP possesses a sufficiently high amount of acidic groups to generate the ion-pair bond with the active ingredient. Further acidification is therefore counterproductive, since too many carboxylate groups would be reprotonated and are thus no longer available for ion-pair bonding.

### In vitro disintegration experiments:

Disintegration was measured for the reference formulation as well as the representative formulations A2 and B2.

Tests were performed with a Pharma Test PTZ Auto 2 EZ testing device using an ODF basket. Test medium was 800 ml demineralized water with a temperature of 37 ± 2 °C.

Formulations were tested with a rectangular shape in a size of 7.04 cm². One side of the formulation was clamped onto the upper side of the ODF basket while a metal clamp was attached on the opposing site of the formulation as a weight. Afterwards the films were brought in the tempered water bath of the testing device and measurement was started. Disintegration time is defined in this test as the time the formulation requires to loose its integrity in such a scale that the film is ripped in pieces and the underside, equipped with the metallic clamp sinks down to the bottom of the testing device closing an electric circuit sending a signal that the test is finished.

For the three formulations the following disintegration times were determined. Tests were performed with n = 3

Reference formulation: mean value 7 s (smallest value 7 s, largest value 8 s).

Formulation A2: mean value 25 s (smallest value 25 s, largest value 25 s).

Formulation B2: mean value 5 s (smallest value 3 s, largest value 6 s).

## Claims

1. Oral thin film comprising a polymer matrix, which comprises an active pharmaceutical ingredient, a first polymer and a second polymer, wherein the first polymer is different from the second polymer and wherein the active pharmaceutical ingredient comprises ulipristal acetate and the second polymer is an anionic.

2. Oral thin film according to claim 1, wherein the first polymer is a water-soluble or water-swellable polymer.

3. Oral thin film according to any of the preceding claims, wherein the first polymer is a water-soluble or water-swellable polymer selected from the group consisting of starch and starch derivatives, dextrans, cellulose derivatives, hydroxypropyl cellulose, hydroxyethyl cellulose, hydroxypropylmethyl cellulose, hydroxypropyl ethyl cellulose, , polyvinylpyrrolidones, polyvinyl alcohols, polyethylene oxide polymers, polyacrylamides, polyethylene glycols, gelatine, collagen, pullulan, tragacanth, arabinogalactan, galactomannan, agar, agarose, carrageenan and/or natural gums.

4. Oral thin film according to any of the preceding claims, wherein the anionic polymer comprises a polymer backbone and anionic groups in side chains, such as sulfones, carboxylates and/or phosphates.

5. Oral thin film according to any of the preceding claims, wherein the anionic polymer comprises polyacrylic acid and/or a carboxylate modified cellulose.

6. Oral thin film according to any of the preceding claims, wherein the first polymer is contained in the polymer matrix in an amount of 10 to 60 wt.-%, preferably 20 to 40 wt.-%, based on the total weight of the polymer matrix.

7. Oral thin film according to any of the preceding claims, wherein the second polymer is contained in the polymer matrix in an amount of 1 to 30 wt.-%, preferably 2 to 15 wt.-%, based on the total weight of the polymer matrix.

8. Oral thin film according to any of the preceding claims, wherein the weight ratio of the first polymer to the second polymer is from 15:1 to 2:1.

9. Oral thin film according to any of the preceding claims, wherein the active pharmaceutical ingredient is contained in the polymer matrix in an amount of 20 to 50 wt.-%, preferably 35 to 45 wt.-%, based on the total weight of the polymer matrix.

10. Method for preparing an oral thin film according to any one of claims 1 to 9, comprising the following steps:
a) preparing a solution, suspension and/or dispersion comprising ulipristal acetate, a first polymer and a second polymer, wherein first polymer is different form the second polymer and wherein the second polymer is an anionic polymer,
b) casting or coating the solution, suspension and/or dispersion obtained in step a) on a support, coating liner or in a mould to spread the suspension, and
c) evaporating the solvent to obtain an oral thin film.

11. Oral thin film obtained by the method according to claim 10.

12. Oral thin film according to any one of claims 1 to 9 or 11 for use as a medicament, in particular for use as an emergency contraceptive.

## Patentansprüche

1. Oraler Dünnfilm, umfassend eine Polymermatrix, die einen pharmazeutischen Wirkstoff, ein erstes Polymer und ein zweites Polymer umfasst, wobei das erste Polymer von dem zweiten Polymer verschieden ist und wobei der pharmazeutische Wirkstoff Ulipristalacetat umfasst und das zweite Polymer ein anionisches ist.

2. Oraler Dünnfilm nach Anspruch 1, wobei das erste Polymer ein wasserlösliches oder wasserquellbares Polymer ist.

3. Oraler Dünnfilm nach einem der vorstehenden Ansprüche, wobei das erste Polymer ein wasserlösliches oder wasserquellbares Polymer ist, das aus der Gruppe, bestehend aus Stärke und Stärkederivaten, Dextranen, Cellulosederivaten, Hydroxypropylcellulose, Hydroxyethylcellulose, Hydroxypropylmethylcellulose, Hydroxypropylethylcellulose, , Polyvinylpyrrolidonen, Polyvinylalkoholen, Polyethylenoxidpolymeren, Polyacrylamiden, Polyethylenglykolen, Gelatine, Kollagen, Pullulan, Traganth, Arabinogalactan, Galactomannan, Agar, Agarose, Carrageen und/oder natürlichen Gummis, ausgewählt ist.

4. Oraler Dünnfilm nach einem der vorstehenden Ansprüche, wobei das anionische Polymer eine Polymerhauptkette und anionische Gruppen, wie etwa Sulfone, Carboxylate und/oder Phosphate, in Seitenketten umfasst.

5. Oraler Dünnfilm nach einem der vorstehenden Ansprüche, wobei das anionische Polymer Polyacrylsäure und/oder eine carboxylatmodifizierte Cellulose umfasst.

6. Oraler Dünnfilm nach einem der vorstehenden Ansprüche, wobei das erste Polymer in der Polymermatrix in einer Menge von 10 bis 60 Gew.-%, vorzugsweise 20 bis 40 Gew.-%, basierend auf dem Gesamtgewicht der Polymermatrix, enthalten ist.

7. Oraler Dünnfilm nach einem der vorstehenden Ansprüche, wobei das zweite Polymer in der Polymermatrix in einer Menge von 1 bis 30 Gew.-%, vorzugsweise 2 bis 15 Gew.-%, basierend auf dem Gesamtgewicht der Polymermatrix, enthalten ist.

8. Oraler Dünnfilm nach einem der vorstehenden Ansprüche, wobei das Gewichtsverhältnis des ersten Polymers zu dem zweiten Polymer 15:1 bis 2:1 beträgt.

9. Oraler Dünnfilm nach einem der vorstehenden Ansprüche, wobei der pharmazeutische Wirkstoff in der Polymermatrix in einer Menge von 20 bis 50 Gew.-%, vorzugsweise 35 bis 45 Gew.-%, basierend auf dem Gesamtgewicht der Polymermatrix, enthalten ist.

10. Verfahren zum Herstellen eines oralen Dünnfilms nach einem der Ansprüche 1 bis 9, umfassend die folgenden Schritte:
a) Herstellen einer Lösung, Suspension und/oder Dispersion, umfassend Ulipristalacetat, ein erstes Polymer und ein zweites Polymer, wobei das erste Polymer von dem zweiten Polymer verschieden ist und das zweite Polymer ein anionisches Polymer ist,
b) Gießen oder Beschichten der in Schritt a) erhaltenen Lösung, Suspension und/oder Dispersion auf einen Träger, eine Beschichtungslage oder in eine Form, um die Suspension zu verteilen, und
c) Verdampfen des Lösungsmittels, um einen oralen Dünnfilm zu erhalten.

11. Oraler Dünnfilm, erhalten durch das Verfahren nach Anspruch 10.

12. Orale Dünnfilm nach einem der Ansprüche 1 bis 9 oder 11 zur Verwendung als Medikament, insbesondere zur Verwendung als Notfallkontrazeptivum.

## Revendications

1. Film mince oral comprenant une matrice polymère, qui comprend un ingrédient pharmaceutique actif, un premier polymère et un second polymère, dans lequel le premier polymère est différent du second polymère et dans lequel l'ingrédient pharmaceutique actif comprend de l'acétate d'ulipristal et le second polymère est un anionique.

2. Film mince oral selon la revendication 1, dans lequel le premier polymère est un polymère soluble dans l'eau ou gonflable à l'eau.

3. Film mince oral selon l'une quelconque des revendications précédentes, dans lequel le premier polymère est un polymère soluble dans l'eau ou gonflable à l'eau choisi dans le groupe consistant en amidon et dérivés d'amidon, dextrans, dérivés de cellulose, hydroxypropyl cellulose, hydroxyéthyl cellulose, hydroxypropylméthyl cellulose, hydroxypropyl éthyl cellulose, polyvinylpyrrolidones, alcools polyvinyliques, polymères d'oxyde de polyéthylène, polyacrylamides, polyéthylène glycols, gélatine, collagène, pullulane, tragacanth, arabinogalactane, galactomannane, gélose, agarose, carraghénane et/ou gommes naturelles.

4. Film mince oral selon l'une quelconque des revendications précédentes, dans lequel le polymère anionique comprend un squelette polymère et des groupes anioniques en chaînes latérales, tels que les sulfones, carboxylates et/ou phosphates.

5. Film mince oral selon l'une quelconque des revendications précédentes, dans lequel le polymère anionique comprend de l'acide polyacrylique et/ou une cellulose modifiée par carboxylate.

6. Film mince oral selon l'une quelconque des revendications précédentes, dans lequel le premier polymère est contenu dans la matrice polymère en une quantité de 10 à 60 % en poids, de préférence 20 à 40 % en poids, par rapport au poids total de la matrice polymère.

7. Film mince oral selon l'une quelconque des revendications précédentes, dans lequel le second polymère est contenu dans la matrice polymère en une quantité de 1 à 30 % en poids, de préférence 2 à 15 % en poids, par rapport au poids total de la matrice polymère.

8. Film mince oral selon l'une quelconque des revendications précédentes, dans lequel le rapport pondéral du premier polymère au second polymère est de 15:1 à 2:1.

9. Film mince oral selon l'une quelconque des revendications précédentes, dans lequel l'ingrédient pharmaceutique actif est contenu dans la matrice polymère en une quantité de 20 à 50 % en poids, de préférence 35 à 45 % en poids, par rapport au poids total de la matrice polymère.

10. Procédé pour préparer un film mince oral selon l'une quelconque des revendications 1 à 9, comprenant les étapes suivantes :
a) la préparation d'une solution, suspension et/ou dispersion comprenant de l'acétate d'ulipristal, un premier polymère et un second polymère, dans lequel le premier polymère est différent du second polymère et dans lequel le second polymère est un polymère anionique,
b) la coulée ou l'enduction de la solution, suspension et/ou dispersion obtenue à l'étape a) sur un support, revêtement ou dans un moule pour étaler la suspension, et
c) l'évaporation du solvant pour obtenir un film mince oral.

11. Film mince oral obtenu par le procédé selon la revendication 10.

12. Film mince oral selon l'une quelconque des revendications 1 à 9 ou 11 pour une utilisation en tant que médicament, en particulier pour une utilisation en tant que contraception d'urgence.
